# EUROPEAN PATENT APPLICATION

(11) **EP 2 548 570 A1**
(43) Date of publication of application: **23.01.2013**
(21) Application number: 11305939.8
(22) Date of filing: 19.07.2011
(51) Int. Cl.: A61K 38/18, A61K 38/26, A61K 31/155, A61P 3/10

(54) **Pharmaceutical composition for treating a metabolic syndrome**

(71) Applicant: SANOFI, 75008 Paris (FR)
(72) Inventor: The designation of the inventor has not yet been filed
(74) Representative: Schneider, Rudolf

(57) **Abstract**

The invention is directed to a pharmaceutical composition comprising at least one FGF-21 (fibroblast growth factor 21) compound, at least one GLP-1R (glucagon-like peptide-1 receptor) agonist and optionally at least one anti-diabetic drug and/or at least one DPP-4 (dipeptidyl peptidase-4) inhibitor for the treatment of at least one metabolic syndrome and/or atherosclerosis, in particular diabetes, dyslipidemia, obesity and/or adipositas. The invention is also directed to a pharmaceutical composition comprising at least one FGF-21 (fibroblast growth factor 21) compound, at least one DPP-4 (dipeptidyl peptidase-4) inhibitor and optionally GLP-1 R (glucagon-like peptide-1 receptor) agonist and/or at least one at least one anti-diabetic drug for the treatment of at least one metabolic syndrome and/or atherosclerosis, in particular diabetes, dyslipidemia, obesity and/or adipositas.

## Description

The present invention is directed to a pharmaceutical composition comprising at least one FGF-21 (fibroblast growth factor 21) compound, at least one GLP-1R (glucagon-like peptide-1 receptor) agonist and optionally at least one anti-diabetic drug and/or at least one DPP-4 (dipeptidyl peptidase-4) inhibitor for the treatment of at least one metabolic syndrome and/or atherosclerosis, in particular diabetes, dyslipidemia, obesity and/or adipositas. Moreover, the present invention is directed to a pharmaceutical composition comprising at least one FGF-21 (fibroblast growth factor 21) compound, at least one DPP-4 (dipeptidyl peptidase-4) inhibitor and optionally at least one GLP-1 R (glucagon-like peptide-1 receptor) agonist and/or at least one anti-diabetic drug for the treatment of at least one metabolic syndrome and/or atherosclerosis, in particular diabetes, dyslipidemia, obesity and/or adipositas.

### Background

Diabetes mellitus is characterized by its clinical manifestations, namely the non-insulin-dependent or maturity onset form, also known as Type 2 diabetes and the insulin-dependent or juvenile onset form, also known as Type 1 diabetes. The manifestations of clinical symptoms of Type 2 diabetes and the underlying obesity usually appear at an age over 40. In contrast, Type 1 diabetes usually shows a rapid onset of the disease often before 30. The disease is a metabolic disorder in humans with a prevalence of approximately one percent in the general population, with one-fourth of these being Type 1 and three-fourth of these being Type 2 diabetes. Type 2 diabetes is a disease characterized by high-circulating blood glucose, insulin and corticosteroid levels.

Currently, there are various pharmacological approaches for the treatment of Type 2 diabetes, which may be utilized individually or in combination, and which act via different modes of action:
1) sulfonylurea stimulate insulin secretion;
2) biguanides (metformin) act by promoting glucose utilization, reducing hepatic glucose production and diminishing intestinal glucose output;
3) oc-glucosidase inhibitors (acarbose, miglitol) slow down carbohydrate digestion and consequently absorption from the gut and reduce postprandial hyperglycemia;
4) thiazolidinediones (troglitazone) enhance insulin action, thus promoting glucose utilization in peripheral tissues; and
5) insulin stimulates tissue glucose utilization and inhibits hepatic glucose output.

However, most of the drugs have limited efficacy and do not address the most important problems, the declining β-cell function and the associated obesity.

Obesity is a chronic disease that is highly prevalent in modern society and is associated with numerous medical problems including diabetes mellitus, insulin resistance, hypertension, hypercholesterolemia, and coronary heart disease. It is further highly correlated with diabetes and insulin resistance, the latter of which is generally accompanied by hyperinsulinemia or hyperglycemia, or both. In addition, Type 2 diabetes is associated with a two to fourfold risk of coronary artery disease.

Type 1 diabetics characteristically show very low or immeasurable plasma insulin with elevated glucagon. An immune response specifically directed against β-cells leads to Type 1 diabetes because β-cells secrete insulin. Current therapeutic regimens for Type 1 diabetes try to minimize hyperglycemia resulting from the lack of natural insulin.

Fibroblast growth factor 21 (FGF21) is a novel metabolic regulator produced primarily by the liver that exerts potent antidiabetic and lipid-lowering effects in animal models of obesity and type 2 diabetes mellitus. This hormone contributes to body weight regulation and is involved in the response to nutritional deprivation and ketogenic state in mice. The principal sites of metabolic actions of FGF21 are adipose tissue, liver and pancreas. Experimental studies have shown improvements in diabetes compensation and dyslipidemia after FGF21 administration in diabetic mice and primates (Dostalova *et al.* 2009). FGF21 has been shown to stimulate glucose uptake in mouse 3T3-L1 adipocytes in the presence and absence of insulin, and to decrease fed and fasting blood glucose, triglycerides, and glucagon levels in ob/ob and db/db mice and 8 week olf ZDF rats in a dose dependant manner, thus, providing the basis for the use of FGF-21 as a therapy for treating diabetes and obesity (see e.g. WO03/011213).

Fibroblast growth factors (FGFs) are polypeptides widely expressed in developing and adult tissues. The FGF family currently consists of twenty-two members, FGF-1 to FGF-23. The members of the FGF family are highly conserved in both gene structure and amino acid sequence between vertebrate species. There are 18 mammalian fibroblast growth factors (FGF1-FGF10 and FGF16-FGF23) which are grouped into 6 subfamilies based on differences in sequence homology and phylogeny. The numbered 'FGFs' that are unassigned to subfamilies - the FGF homologous factors (previously known as FGF11-FGF14) - have high sequence identity with the FGF family but do not activate FGF receptors (FGFRs) and are therefore not generally considered members of the FGF family.

While most of FGFs act as local regulators of cell growth and differentiation, recent studies indicated that FGF19 subfamily members including FGF15/19, FGF21 and FGF23 exert important metabolic effects by an endocrine fashion. The members of FGF19 subfamily regulate diverse physiological processes that are not affected by classical FGFs. The wide variety of metabolic activities of these endocrine factors include the regulation of the bile acid, carbohydrate and lipid metabolism as well as phosphate, calcium and vitamin D homeostasis (Tomlinson *et al.* 2002, Holt *et al.* 2003, Shimada *et al.* 2004, Kharitonenkov *et al.* 2005, Inagaki *et al.* 2005, Lundasen *et al.* 2006).

FGF21 was originally isolated from mouse embryos. FGF21 mRNA was most abundantly expressed in the liver, and to lesser extent in the thymus (Nishimura *et al.* 2000). Human FGF21 is highly identical (approximately 75 % amino acid identity) to mouse FGF21. Among human FGF family members, FGF21 is the most similar (approximately 35 % amino acid identity) to FGF19 (Nishimura *et al.* 2000). FGF21 is free of the proliferative and tumorigenic effects (Kharitonenkov *et al.* 2005, Huang *et al.* 2006, Wente *et al.* 2006) that are typical for majority of the members of FGF family (Ornitz and Itoh 2001, Nicholes *et al.* 2002, Eswarakumar *et al.* 2005).

The administration of FGF21 to obese leptin-deficient ob/ob and leptin receptor-deficient *db*/*db* mice and obese ZDF rats significantly lowered blood glucose and triglycerides, decreased fasting insulin levels and improved glucose clearance during an oral glucose tolerance test. FGF21 did not affect food intake or body weight/composition of diabetic or lean mice and rats over the course of 2 weeks of administration. Importantly, FGF21 did not induce mitogenicity, hypoglycemia, or weight gain at any dose tested in diabetic or healthy animals or when overexpressed in transgenic mice (Kharitonenkov *et al.* 2005). FGF21-overexpressing transgenic mice were resistant to diet-induced obesity. The administration of FGF21 to diabetic rhesus monkeys for 6 weeks reduced fasting plasma glucose, fructosamine, triglyceride, insulin and glucagone levels. Importantly, hypoglycemia was not observed during the study despite of significant glucose-lowering effects. FGF21 administration also significantly lowered LDL-cholesterol and increased HDL-cholesterol and, in contrast to mice (Kharitonenkov *et al.* 2005), slightly but significantly decreased body weight (Kharitonenkov *et al.* 2007).

Further information can be taken from the following references:
1. DOSTALOVA I. et al.: Fibroblast Growth Factor 21: A Novel Metabolic Regulator With Potential Therapeutic Properties in Obesity/Type 2 Diabetes Mellitus. Physiol Res 58: 1-7, 2009.
2. ESWARAKUMAR V.P. et al.: Cellular signaling by fibroblast growth factor receptors. Cytokine Growth Factor Rev 16: 139-149, 2005.
3. HOLT J.A. et al.: Definition of a novel growth factor-dependent signal cascade for the suppression of bile acid biosynthesis. Genes Dev 17: 1581-1591, 2003.
4. HUANG X. et al.: Forced expression of hepatocytespecific fibroblast growth factor 21 delays initiation of chemically induced hepatocarcinogenesis. Mol Carcinog 45: 934-942, 2006.
5. INAGAKI T. et al.: Endocrine regulation of the fasting response by PPARα-mediated induction of fibroblast growth factor 21. Cell Metab 5: 415-425, 2007.
6. KHARITONENKOV A. et al.: FGF-21 as a novel metabolic regulator. J Clin Invest 115: 1627-1635, 2005.
7. KHARITONENKOV A. et al.: The metabolic state of diabetic monkeys is regulated by fibroblast growth factor-21. Endocrinology 148: 774-781, 2007.
8. LUNDASEN T. et al.: Circulating intestinal fibroblast growth factor 19 has a pronounced diurnal variation and modulates hepatic bile acid synthesis in man. J Intern Med 260: 530-536, 2006.
9. NICHOLES K. et al.: A mouse model of hepatocellular carcinoma: ectopic expression of fibroblast growth factor 19 in skeletal muscle of transgenic mice. Am J Pathol 160: 2295-2307, 2002.
10. NISHIMURA T. et al.: Identification of a novel FGF, FGF-21, preferentially expressed in the liver. Biochim Biophys Acta 1492: 203-206, 2000.
11. ORNITZ D.M. et al.: Fibroblast growth factors. Genome Biol 2: REVIEWS3005, 2001.
12. SHIMADA T. et al.: FGF-23 is a potent regulator of vitamin D metabolism and phosphate homeostasis. J Bone Miner Res 19: 429-435, 2004.
13. TOMLINSON E. et al.: Transgenic mice expressing human fibroblast growth factor-19 display increased metabolic rate and decreased adiposity. Endocrinology 143: 1741-1747, 2002.
14. WENTE W. et al.: Fibroblast growth factor-21 improves pancreatic beta-cell function and survival by activation of extracellular signal-regulated kinase 1/2 and Akt signaling pathways. Diabetes 55: 2470-2478, 2006.

The gut peptide glucagon-like peptide-1 (GLP-1) is an incretin hormone and secreted in a nutrient-dependent manner. It stimulates glucose-dependent insulin secretion. GLP-1 also promotes β-cell proliferation and controls glycemia via additional actions on glucose sensors, inhibition of gastric emptying, food intake and glucagons secretion. Furthermore, GLP-1 stimulates insulin secretion and reduces blood glucose in human subjects with Type 2 diabetes. Exogenous administration of bioactive GLP-1, GLP-1 (7-27) or GLP-1 (7-36 amide), in doses elevating plasma concentrations to approximately 3-4 fold physiological postprandial levels fully normalizes fasting hyperglycaemia in Type 2 diabetic patients (Nauck, M. A. et al. (1997) Exp Clin Endocrinol Diabetes, 105, 187-197). The human GLP-1 receptor (GLP-1 R) is a 463 amino acid heptahelical G protein-coupled receptor widely expressed in pancreatic islets, kidney, lung, heart and multiple regions of the peripheral and central nervous system. Within islets, the GLP-1 R is predominantly localized to islet β-cells. Activation of GLP-1R signalling initiates a program of differentiation toward a more endocrine-like phenotype, in particular the differentiation of progenitors derived from human islets into functioning β-cells (Drucker, D. J. (2006) Cell Metabolism, 3, 153-165).

Unfortunately, both, FGF-21 and bioactive GLP-1, as well as other known drugs have limited efficacy by themselves to the complex and multifactorial metabolic dysfunctions which can be observed in Type 2 diabetes or other metabolic disorders. This applies also for the efficacy in lowering the blood glucose levels by said compounds themselves. According to the present invention it has surprisingly been found that the combination of FGF-21 and a GLP-1R agonist significantly lowered blood glucose levels in a synergistic manner up to normo-glycaemic levels. Moreover it has surprisingly been found that the combination of FGF-21 and a DPP-IV inhibitor significantly lowered hepatic lipid levels in a synergistic manner

### Embodiments

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. The same applies to the term "includes" and variations thereof such as "including" and "inclusion".

One embodiment of the present invention is, therefore, directed to a pharmaceutical composition comprising at least one FGF-21 (fibroblast growth factor 21) compound and at least one GLP-1 R (glucagon-like peptide-1 receptor) agonist.

The term "pharmaceutical composition" as used herein includes (but is not limited to) the formulation of the active compound with a carrier. The carrier can e.g. be an encapsulating material providing a capsule in which the active component(s)/ingredient(s) with or without other carriers, is surrounded by a carrier, which is thus, in association with it. The carrier can also be suitable for a liquid formulation of the active ingredient(s), and preferably be itself a liquid. The carrier can also be any other carrier as suitable for the intended formulation of the pharmaceutical composition.

"Pharmaceutically acceptable" means approved by a regulatory agency of the Federal or a state government or a supra-national organisation of states such as the European Eunion or an economic area such as the European Economic Area or listed in the U.S. Pharmacopeia or other generally recognized pharmacopeia in a given country or economic area for use in animals, and more particularly in humans.

The term "carrier", as used herein, refers to a pharmacologically inactive substance such as but not limited to a diluent, excipient, or vehicle with which the therapeutically active ingredient is administered. Such pharmaceutical carriers can be liquid or solid. Liquid carrier include but are not limited to sterile liquids, such as saline solutions in water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. A saline solution is a preferred carrier when the pharmaceutical composition is administered intravenously.

Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E. W. Martin.

The term "active ingredient" refers to the substance in a pharmaceutical composition or formulation that is biologically active, i.e. that provides pharmaceutical value. A pharmaceutical composition may comprise one or more active ingredients which may act in conjunction with or independently of each other.

The active ingredient can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as but not limited to those derived from sodium, potassium, ammonium, calcium, ferric hydroxides, isopropylamine, triethylamine, 2-ethylamino ethanol, histidine, procaine, and the like.

As used herein, a "patient" means any mammal, reptile or bird that may benefit from a treatment with a pharmaceutical composition as described herein. Preferably, a "patient" is selected from the group consisting of laboratory animals (e.g. monkey, mouse or rat), domestic animals (including e.g. guinea pig, rabbit, horse, donkey, cow, sheep, goat, pig, chicken, camel, cat, dog, turtle, tortoise, snake, or lizard), or primates including chimpanzees, bonobos, gorillas and human beings. It is particularly preferred that the "patient" is a human being.

As used herein, "treat", "treating" or "treatment" of a disease or disorder means accomplishing one or more of the following: (a) reducing the severity of the disorder; (b) limiting or preventing development of symptoms characteristic of the disorder(s) being treated; (c) inhibiting worsening of symptoms characteristic of the disorder(s) being treated; (d) limiting or preventing recurrence of the disorder(s) in patients that have previously had the disorder(s); and (e) limiting or preventing recurrence of symptoms in patients that were previously symptomatic for the disorder(s).

As used herein, "administering" includes *in vivo* administration, as well as administration directly to tissue *ex vivo,* such as vein grafts.

An "effective amount" is an amount of a therapeutic agent sufficient to achieve the intended purpose. The effective amount of a given therapeutic agent will vary with factors such as the nature of the agent, the route of administration, the size and species of the animal to receive the therapeutic agent, and the purpose of the administration. The effective amount in each individual case may be determined empirically by a skilled artisan according to established methods in the art.

A "FGF-21 compound" is defined as a compound showing FGF-21 activity, in particular comprising (i) native FGF-21 or (ii) a FGF-21 mimetic with FGF-21 activity.

The term "native FGF-21" refers to the naturally occurring FGF-21 or a variant being substantially homologous to native FGF-21. Typically, such FGF-21 variant is biologically equivalent to native FGF-21, i.e. is capable of exhibiting all or some properties in an identical or similar manner as naturally occurring FGF-21. In preferred embodiments the native FGF-21 is mammalian FGF-21, preferably selected from the group consisting of mouse, rat, rabbit, sheep, cow, dog, cat, horse, pig, monkey, and human FGF-21. Human FGF-21 as shown in SEQ ID NO: 1 is particularly preferred.

A variant being "substantially homologous" to native FGF-21 is characterized by a certain degree of sequence identity to native FGF-21 from which it is derived. More precisely, in the context of the present invention, a variant being substantially homologous to native FGF-21 exhibits at least 80% sequence identity to native FGF-21.

The term "at least 80% sequence identity" is used throughout the specification with regard to polypeptide sequence comparisons. This expression preferably refers to a sequence identity of at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% to the respective reference polypeptide. FGF-21 variants may additionally or alternatively comprise deletions of amino acids, which may be N-terminal truncations, C-terminal truncations or internal deletions or any combination of these. Such variants comprising N-terminal truncations, C-terminal truncations and/or internal deletions are referred to as "deletion variant" or "fragments" in the context of the present application. The terms "deletion variant" and "fragment" are used interchangeably herein. A fragment may be naturally occurring (e.g. splice variants) or it may be constructed artificially, preferably by gene-technological means. Preferably, a fragment (or deletion variant) has a deletion of up to 1, 2, 3, 4, 5, 6, 7, 8, 9, 10, 15, 20, 25, 30, 35, 40, 45, 50, 55, 60, 65, 70, 75, 80, 85, 90, 95, or 100 amino acids at its N-terminus and/or at its C-terminus and/or internally as compared to the parent polypeptide, preferably at its N-terminus, at its N- and C-terminus, or at its C-terminus. In case where two sequences are compared and the reference sequence is not specified in comparison to which the sequence identity percentage is to be calculated, the sequence identity is to be calculated with reference to the longer of the two sequences to be compared, if not specifically indicated otherwise. If the reference sequence is indicated, the sequence identity is determined on the basis of the full length of the reference sequence indicated by SEQ ID, if not specifically indicated otherwise. For example, a peptide sequence consisting of 105 amino acids compared to the amino acid sequence of FGF-21 according to SEQ ID NO: 1 may exhibit a maximum sequence identity percentage of 50,24% (105/209) while a sequence with a length of 181 amino acids may exhibit a maximum sequence identity percentage of 86.6% (181/209).

The similarity of amino acid sequences, i.e. the percentage of sequence identity, can be determined via sequence alignments. Such alignments can be carried out with several art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on http://www.ebi.ac.uk/Tools/clustalw/ or on http://www.ebi.ac.uk/Tools/clustalw2/index.html or on http://npsa-pbil.ibcp.fr/cgi-bin/npsa_automat.pl?page=/NPSA/npsa_clustalw.html. Preferred parameters used are the default parameters as they are set on http://www.ebi.ac.uk/Tools/clustalw/ or http://www.ebi.ac.uk/Tools/clustalw2/index.html. The grade of sequence identity (sequence matching) may be calculated using e.g. BLAST, BLAT or BlastZ (or BlastX). A similar algorithm is incorporated into the BLASTN and BLASTP programs of Altschul et al. (1990) J. Mol. Biol. 215: 403-410. BLAST polynucleotide searches are performed with the BLASTN program, score = 100, word length = 12, to obtain polynucleotide sequences that are homologous to those nucleic acids which encode F, N, or M2-1. BLAST protein searches are performed with the BLASTP program, score = 50, word length = 3, to obtain amino acid sequences homologous to the F polypeptide, N polypeptide, or M2-1 polypeptide. To obtain gapped alignments for comparative purposes, Gapped BLAST is utilized as described in Altschul et al. (1997) Nucleic Acids Res. 25: 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1:I54-I62) or Markov random fields. When percentages of sequence identity are referred to in the present application, these percentages are calculated in relation to the full length of the longer sequence, if not specifically indicated otherwise.

FGF-21 mimetics with FGF-21 activity comprise FGF-21 molecules carrying alterations to the amino acid chain of native FGF-21 such that they exhibit FGF-21 activity and further exhibit additional properties such as but not limited to modified chemical properties and/or a prolonged serum half-life. FGF-21 mimetics include but are not limited to FGF-21 muteins, FGF-21 fusion proteins and FGF-21 conjugates.

The term "FGF-21 activity" refers to any known biological activity of naturally occuring FGF-21, such as but not limited to those listed above and in the following:
1) The stimulation of glucose uptake (e.g. in adipocytes such as human or mouse adipocytes, e.g. mouse 3T3-L1 adipocytes) in the presence of insulin and absence of insulin.
2) The increase in glucose-induced insulin secretion from diabetic islets (e.g. from diabetic patients or diabetic test animals such as diabetic rodents or from isolated beta cells from diabetic test animals such as diabetic rodents or isolated islets from diabetic test animals such as diabetic rodents).
3) The decrease of fed and fasting blood glucose levels (e.g. in ob/ob mice, in db/db mice or in 8 week olf ZDF rats in a dose-dependant manner).
4) The decrease of fed and fasting triglycerides (e.g. in ob/ob mice, in db/db mice or in 8 week olf ZDF rats in a dose-dependant manner).
5) The decrease of fed and fasting glucagon levels (e.g. in ob/ob mice, in db/db mice or in 8 week olf ZDF rats in a dose-dependant manner).
6) A lowering of ldl lipoprotein cholesterol and/or raising of hdl lipoprotein cholesterol.
7) An increase in Glut-1 steady state level.
8) The interaction with other proteins, such as FGF-Receptor, especially FGF-Receptor 1, 2 or 3 or a part thereof able to interacti with FGF-21.
9) The activation of certain signaling pathways, e.g. activation of extracellular signal-related kinase 1/2, activation of the Akt signaling pathway.

The term "FGF-21 activity" also refers to the combination of two or more of any of the above-liste activities and also to a combination of one or more of them with any other known beneficial activity of FGF-21.

"FGF-21 activity" can for example be measured in a FGF-21 activity assay generally known to a person skilled in the art. An FGF-21 activity assay is e.g. a "glucose uptake assay" as described in Kharitonenkov, A. et al. (2005), 115; 1627, No. 6. As an example for the glucose uptake assay, adipocytes are starved for 3 hours in DMEM/0.1% BSA, stimulated with FGF-21 for 24 hours, and washed twice with KRP buffer (15 mM HEPES, pH 7.4, 118 mM NaCl, 4.8 mM KCI, 1.2 mM MgSO₄, 1.3 mM CaCl₂, 1.2 mM KH₂PO₄, 0.1% BSA), and 100 µl of KRP buffer containing 2-deoxy-D-[¹⁴C]glucose (2-DOG) (0.1 µCi, 100 µM) is added to each well. Control wells contains 100 µl of KRP buffer with 2-DOG (0.1 µCi, 10 mM) to monitor for nonspecificity. The uptake reaction is carried out for 1 hour at 37°C, terminated by addition of cytochalasin B (20 µM), and measured using Wallac 1450 MicroBeta counter (PerkinElmer, USA).

Examples of FGF-21 mimetics are (a) proteins having at least about 96%, in particular 99% amino acid sequence identity to the amino acid sequence shown in SEQ ID NO: 1 and having FGF-21 activity, (b) a FGF-21 fusion protein or a (c) FGF-21 conjugate, e.g. a FGF-21 mutein, a FGF-21-Fc fusion protein, a FGF-21-HSA fusion protein or a PEGylated FGF-21.

"Muteins" typically comprise alterations such as but not limited to amino acid exchanges, additions and/or deletions to the FGF-21 amino acid chain which maintain the FGF-21 activity and typically alter the chemical properties of the amino acid chain, such as but not limited to an increased or decreased glycosylation or amination of the amino acid chain, and/or an increased or decreased potential to be proteolytically degraded and/or an alteration to the electrostatic surface potential of the protein.

Examples of FGF-21 muteins are described in e.g. WO2005/061712, WO2006/028595, WO2006/028714, WO2006/065582 or WO2008/121563. Exemplary muteins are muteins which have a reduced capacity for O-glycosylation when e.g. expressed in yeast compared to wild-type human FGF-21, e.g. human FGF-21 with a substitution at position 167 (serine), e.g. human FGF-21 with one of the following substitutions: Ser167Ala, Ser167Glu, Ser167Asp, Ser167Asn, Ser167Gln, Ser167Gly, Ser167Val, Ser167His, Ser167Lys or Ser167Tyr. Another example is a mutein which shows reduced deamidation compared to wild-type human FGF-21, e.g. a mutein with a substitution at position 121 (asparagine) of human FGF-21, e.g. Asn121Ala, Asn121Val, Asn121Ser, Asn121Asp or Asn121Glu. An alternative mutein is human FGF-21 having one or more non-naturally encoded amino acids, e.g. as described by the general formula in claim 29 of WO2008/121563. Other muteins comprise a substitution of a charged (e.g. aspartate, glutamate) or polar but uncharged amino acids (e.g. serine, threonine, asparagine, glutamine) for e.g. a polar but uncharged or charged amino acid, respectively. Examples are Leu139Glu, Ala145Glu, Leu146Glu, Ile152Glu, Gln156Glu, Ser163Glu, Ile152Glu, Ser163Glu or Gln54Glu. Another mutein is a mutein showing a reduced susceptibility for proteolytic degradation when expressed in e.g. yeast compared to human FGF-21, in particular human FGF-21 with a substitution of Leu153 with an amino acid selected from Gly, Ala, Val, Pro, Phe, Tyr, Trp, Ser, Thr, Asn, Asp, Gln, Glu, Cys or Met. A preferred FGF-21 mutein is the mutated FGF-21 according to SEQ ID NO: 2 which carries a deletion of amino acids 1-28 of human FGF-21 (SEQ ID NO: 1) and contains an additional glycine at the N-terminus.

As used herein, the term "fusion protein" refers to the amino acid chain of native FGF-21 or substantially homologous variants of FGF-21 that comprise one or more further amino acid chains. Each amino acid chain is preferably a complete protein, i.e. spanning an entire ORF, or a fragment, domain or epitope thereof. The individual parts of a fusion protein may either be permanently or temporarily connected to each other. Parts of a fusion protein that are permanently connected are translated from a single ORF and are not later separated co- or post-translationally. Parts of fusion proteins that are connected temporarily may also derive from a single ORF but are divided co-translationally due to separation during the translation process or post-translationally due to cleavage of the peptide chain, e.g. by an endopeptidase. Additionally or alternatively, parts of a fusion protein may also be derived from two different ORF and are connected post-translationally, for instance through covalent bonds.

Examples of FGF-21 fusion proteins are described in e.g. WO2004/110472 or WO2005/113606, for example a FGF-21-Fc fusion protein or a FGF-21-HAS fusion protein. "Fc" means the Fc portion of an immunoglobulin, e.g. the Fc portion of lgG4. "HSA" means human serum albumin. Such FGF-21 fusion proteins typically show an extended time of action such as but not limited to an extended serum half-life, compared to native FGF-21 or a substantially homologous variant thereof.

The term "conjugates" as used herein refers to the amino acid chain of native FGF-21 or substantially homologous variants of FGF-21 that comprise alterations of the amino acid chain allowing for chemical conjugations of the amino acid chain such as but not limited to PEGylation, HESylation, or Polysialylation. Such FGF-21 conjugates typically show an extended time of action such as but not limited to an extended serum half-life, compared to native FGF-21 or a substantially homologous variant thereof.

Examples of FGF-21 conjugates are described in e.g. WO2005/091944, WO2006/050247 or WO2009/089396, for example glycol-linked FGF-21 compounds. Such glycol-linked FGF21 compounds usually carry a polyethylene glycol (PEG), e.g. at a cysteine or lysine amino acid residue or at an introduced N-linked or O-linked glycosylation site, (herein referred to as "PEGylated FGF-21 "). Such PEGylated FGF-21 compounds generally show an extended time of action compared to human FGF-21. Suitable PEGs have a molecular weight of about 20,000 to 40,000 daltons.

A "GLP-1 R agonist" is defined as a compound which binds to and activates the GLP-1 receptor, like GLP-1 (glucagon-like peptide 1). Physiological actions of GLP-1 and/or of the GLP-1R agonist are described e.g. in Nauck, M. A. et al. (1997) Exp. Clin. Endocrinol. Diabetes, 105, 187-195. These physiological actions in normal subjects, in particular humans, include e.g. glucose-dependent stimulation of insulin secretion, suppression of glucagon secretion, stimulation of (pro)insulin biosynthesis, reduction of food intake, deceleration of gastric emptying and/or equivocal insulin sensitivity. Suitable assays to discover GLP-1 R agonists are described in e.g. Thorkildsen, Chr. et al. (2003), Journal of Pharmacology and Experimental Therapeutics, 307, 490-496; Knudsen, L. B. et al. (2007), PNAS, 104, 937-942, No. 3; Chen, D. et al. (2007), PNAS, 104, 943-948, No. 3; or US2006/0003417 A1 (see e.g. Example 8). In short, in a "receptor binding assay", a purified membrane fraction of eukaryotic cells harbouring e.g. the human recombinant GLP-1 receptor, e.g. CHO, BHK or HEK293 cells, is incubated with the test compound or compounds in the presence of e.g. human GLP-1, e.g. GLP-1 (7-36) amide which is marked with e.g. ¹²⁵I (e.g. 80 kBq/pmol). Usually different concentrations of the test compound or compounds are used and the IC₅₀ values are determined as the concentrations diminishing the specific binding of human GLP-1. In a "receptor functional assay", isolated plasma membranes from eukaryotic cells, as e.g. described above, expressing e.g. the human GLP-1 receptor were prepared and incubated with a test compound. The functional assay is carried out by measuring cAMP as a response to stimulation by the test compound. In a "reporter gene assay", eukaryotic cells, as e.g. described above, expressing e.g. the human GLP-1 receptor and containing e.g. a multiple response element/cAMP response element-driven luciferase reporter plasmid are cultured in the presence of a test compound. cAMP response element-driven luciferase activities are measured as a response to stimulation by the test compound.

Suitable GLP-1 R agonists are selected from a bioactive GLP-1, a GLP-1 analog or a GLP-1 substitute, as e.g. described in Drucker, D. J. (2006) Cell Metabolism, 3, 153-165; Thorkildsen, Chr. (2003; supra); Chen, D. et al. (2007; supra); Knudsen, L. B. et al. (2007; supra); Liu, J. et al. (2007) Neurochem Int., 51, 361-369, No. 6-7; Christensen, M. et al. (2009), Drugs, 12, 503-513; Maida, A. et al. (2008) Endocrinology, 149, 5670-5678, No. 11 and US2006/0003417. Exemplary compounds are GLP-1 (7-37), GLP-1 (7-36)amide, extendin-4, liraglutide, CJC-1131, *albugon, albiglutide,* exenatide, *exenatide-*LAR, oxyntomodulin, lixisenatide, geniproside, *AVE-0010,* a short peptide with GLP-1 R agonistic activity and/or a small organic compound with GLP-1 R agonistic activity.

In detail, Human GLP-1 (7-37) possesses the amino acid sequence of SEQ ID NO: 3. Human GLP-1 (7-36)amide possesses the amino acid sequence of SEQ ID NO: 4. Extendin-4 possesses the amino acid sequence of SEQ ID NO: 5. Exenatide possesses the amino acid sequence of SEQ ID NO: 6 and oxyntomodulin the amino acid sequence of SEQ ID NO: 7. The amino acid sequence of lixisenatide is shown in SEQ ID NO: 8. The structure of lixisenatide is based on exendin-4(1-39) modified C-terminally with six additional lysine residues in order to resist immediate physiological degradation by DPP-4 (dipeptidyl peptidase-4). The amino acid sequence of *AVE0010* is shown in SEQ ID NO: 9

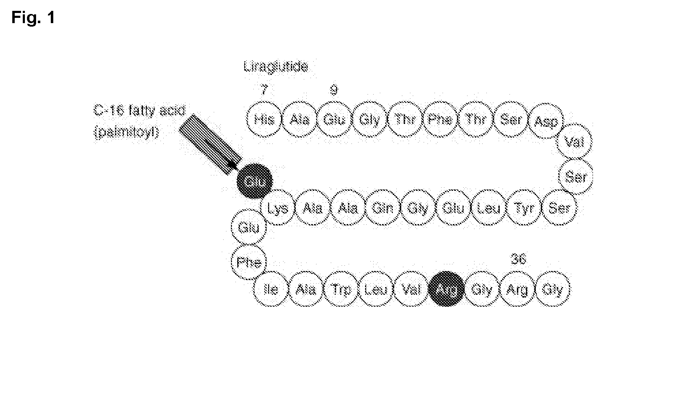

The chemical structure of liraglutide is shown in Fig. 1. Liraglutide was obtained by substitution of Lys 34 of GLP-1 (7-37) to Arg, and by addition of a C16 fatty acid at position 26 using a y-glutamic acid spacer. The chemical name is [N-epsilon(gamma-L-glutamoyl(N-alpha-hexadecanoyl)-Lys²⁶,Arg³⁴-GLP-1(7-37)].

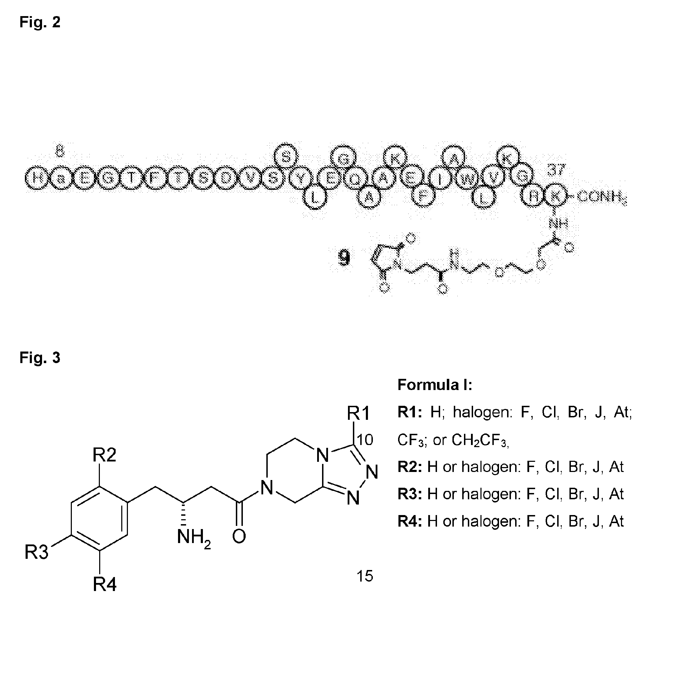

The chemical structure of CJC-1131 is shown in Fig. 2. Albumin is attached at the C-terminal of GLP-1 with a d-alanine substitution at position 8. CJC-1131 shows a very good combination of stability and bioactivity.

Other peptides with GLP-1R agonistic activity are exemplary disclosed in US 2006/0003417 and small organic compound with GLP-1R agonistic activity are exemplary disclosed in Chen et al. 2007, PNAS, 104, 943-948, No. 3 or Knudsen et al., 2007, PNAS, 104, 937-942.

In a further embodiment of the present invention the pharmaceutical composition additionally comprises at least one anti-diabetic drug and/or at least one DPP-4 inhibitor.

As used herein, the term "anti-diabetic drug" refers to pharmaceuticals showing a mode of action reducing the symptoms and/or causes of Diabetes mellitus. Exemplary anti-diabetic drugs are
a) insulin,
b) thiazolidinedione, e.g. rosiglitazone or pioglitazone (see e.g. WO2005/072769), metformin (N,N-dimethylimidodicarbonimidic-diamide), or
c) sulphonylurea, such as chlorpropamide (4-chloro-N-(propylcarbamoyl)-benzenesulfonamide), tolazamide (*N*-[(azepan-1-ylamino)carbonyl]-4-methyl-benzenesulfonamide), gliclazide (N-(hexahydrocyclopenta[*c*]pyrrol-2(1 *H*)-yl-carbamoyl)-4-methylbenzenesulfonamide), or glimepiride (3-ethyl-4-methyl-N-(4-[N-((1r,4r)-4-methylcyclohexylcarbamoyl)-sulfamoyl]phenethyl)-2-oxo-2,5-dihydro-1H-pyrrole-1-carboxamide).

According to the present invention "insulin" means naturally occurring insulin, modified insulin or an insulin analogue, including salts thereof, and combinations thereof, e.g. combinations of a modified insulin and an insulin analogue, for example insulins which have amino acid exchanges/deletions/additions as well as further modifications such as acylation or other chemical modification. One example of this type of compound is insulin detemir, i.e. LysB29-tetradecanoyl/des(B30) human insulin. Another example may be insulins in which unnatural amino acids or amino acids which are normally noncoding in eukaryotes, such as D-amino acids, have been incorporated (Geiger, R. et al., Hoppe Seylers Z. Physiol. Chem. (1976) 357, 1267-1270; Geiger, R. et al., Hoppe Seylers Z. Physiol. Chem. (1975) 356, 1635-1649, No. 10; Krail, G. et al., Hoppe Seylers Z. Physiol. Chem. (1971) 352, 1595-1598, No. 11). Yet other examples are insulin analogues in which the C-terminal carboxylic acid of either the A-chain or the B-chain, or both, are replaced by an amide.

"Modified insulin" is preferably selected from acylated insulin with insulin activity, in particular wherein one or more amino acid(s) in the A and/or B chain of insulin is/are acylated, preferably human insulin acylated at position B29 (Tsai, Y. J. et al. (1997) Journal of Pharmaceutical Sciences, 86, 1264-1268, No. 11). Other acetylated insulins are desB30 human insulin or B01 bovine insulin (Tsai, Y. J. et al., supra). Other Examples of acylated insulin are e.g. disclosed in US 5,750,497 and US 6,011,007. An overview of the structure-activity relationships for modified insulins, is provided in Mayer, J. P. et al. (2007) Biopolymers, 88, 687-713, No. 5. Modified insulins are typically prepared by chemical and/or enzymatic manipulation of insulin, or a suitable insulin precursor such as preproinsulin, proinsulin or truncated analogues thereof.

An "insulin analogue" is preferably selected from insulin with insulin activity having one or more mutation(s), substitution(s), deletion(s) and/or addition(s), in particular an insulin with a C- and/or N-terminal truncation or extension in the A and/or B chain, preferably des(B30) insulin, PheB1 insulin, B1-4 insulin, AspB28 human insulin (insulin aspart), LysB28/ProB29 human insulin (insulin lispro), LysB03/GluB29 human insulin (insulin glulisine) or GlyA21/ArgB31/ArgB32 human insulin (insulin glargine). The only proviso of an insulin analogue is that it has a sufficient insulin activity. An overview of the structure-activity relationships for insulin analogues, with discussion of which amino acid exchanges, deletions and/or additions are tolerated is provided in Mayer, J. P. et al. (2007; supra). The insulin analogues are preferably such wherein one or more of the naturally occurring amino acid residues, preferably one, two or three of them, have been substituted by another amino acid residue. Further examples of insulin analogues are C-terminal truncated derivatives such as des(B30) human insulin; B-chain N-terminal truncated insulin analogues such as des PheB1 insulin or des B1-4 insulin; insulin analogues wherein the A-chain and/or B-chain have an N-terminal extension, including so-called "pre-insulins" where the B-chain has an N-terminal extension; and insulin analogues wherein the A-chain and/or the B-chain have C-terminal extension. For example one or two Arg may be added to position B1. Examples of insulin analogues are described in the following patents and equivalents thereto: US 5,618,913, EP 0 254 516 A2 and EP 0 280 534 A2. An overview of insulin analogues in clinical use is provided in Mayer J. P. et al. (2007, supra). Insulin analogues or their precursors are typically prepared using gene technology techniques well known to those skilled in the art, typically in bacteria or yeast, with subsequent enzymatic or synthetic manipulation if required. Alternatively, insulin analogues can be prepared chemically (Cao, Q. P. et al. (1986) Biol. Chem. Hoppe Seyler, 367, 135-140, No. 2). Examples of specific insulin analogues are insulin aspart (i.e. AspB28 human insulin); insulin lispro (i.e. LysB28, ProB29 human insulin); insulin glulisine (ie. LysB03, GluB29 human insulin); and insulin glargine (i.e. GlyA21, ArgB31, ArgB32 human insulin).

### Exemplary DPP-4 Inhibitors are

The compound of formula I (figure 3), sitagliptin: (R)-4-oxo-4-[3-(trifluoromethyl)-5,6-dihydro[1,2,4]triazolo[4,3-a]-pyrazin-7(8H)-yl]-1-(2,4,5-trifluorophenyl)butan-2-amine, vildagliptin: (*S*)-1-[*N*-(3-hydroxy-1-adamantyl)glycyl]pyrrolidine-2-carbonitrile, saxagliptin: (1*S*,3*S*,5*S*)-2-[(2*S*)-2-amino-2-(3-hydroxy-1-adamantyl)-acetyl]-2-azabicyclo[3.1.0]hexane-3-carbonitrile, linagliptin 8-[(3R)-3-aminopiperidin-1-yl]-7-(but-2-yn-1-yl)-3- methyl-1-[(4-methyl-quinazolin-2-yl)methyl]-3,7-dihydro-1*H*-purine-2,6-dione) adogliptin (2-({6-[(3*R*)-3-aminopiperidin-1-yl]-3-methyl-2,4-dioxo-3,4-dihydropyrimidin-1 (2*H*)-yl}methyl)-benzonitrile, and berberine which is a quaternary ammonium salt from the group of isoquinoline alkaloids found in in the roots, rhizomes, stems, and bark of plants such as *Berberis,* goldenseal (*Hydrastis canadensis*), and *Coptis chinensis.*

In a further embodiment of the present invention the pharmaceutical composition comprises at least one FGF-21 (fibroblast growth factor 21) compound and at least one DPP-4 (dipeptidyl peptidase-4) inhibitor. Such composition may additionally comprise at least one anti-diabetic drug and/or at least one GLP1R (glucagon-like peptide-1 receptor) agonist.

A pharmaceutical composition comprising at least one FGF-21 compound (such as naturally occuring FGF-21 (e.g. comprising or having the sequence of SEQ ID NO:1) or an FGF-21 mimetic (e.g. comprising or having the SEQ ID NO.2)) and at least one DPP-4 inhibitor (such as a compound with the formula I, especially sitagliptin) and at least one GLP1 R agonist (such as AVE0010) would also be advantageous as it would combine the synergystic effects of FGF-21 with the GLP1 R-agonist on lowering of blood glucose and the synergistic effects of FGF-21 with the DPPIV inhibitor on lowering hepatic or blood lipids (especially triglycerides). Such a combination might be advantageous for use in the decrease of (plasma, blood and/or hepatic) lipids, preferably raised lipids and especially raised triglycerides (e.g. for application in the treatment of hyperlipidemia or fatty liver disease) and the lowering of blood or plasma glucose levels or increase of glucose tolerance (e.g. for application in the treatment of hyperglycemia, impaired glucose tolerance or diabetes mellitus)), or any of the other combined uses of the three components.

The individual compounds of the pharmaceutical composition of the present invention can be combined in one formulation or contained in several formulations for e.g. simultaneous or subsequent, i.e. sequential administration(s), or combinations thereof.

According to the present invention the combination of at least one FGF-21 compound and at least one GLP-1 R agonist, surprisingly resulted in a synergistic effect in lowering plasma glucose levels as shown with the animal models in the Examples. Moreover, the combination of at least one FGF-21 compound at least one DPP-4 inhibitor surprisingly resulted in a synergistic effect in lowering triglycerides, especially liver triglycerides as shown with the animal models in the examples. The animal models are an ob/ob or obese mouse, a db/db mouse and a DIO mouse (diet-induced-obese mouse). The ob/ob mouse is a mutant mouse which cannot produce the hormone leptin which regulates the appetite. Consequently, the ob/ob mouse eats excessively and becomes profoundly obese. It is a standard animal model for hyperglycemia, insulin resistance and obesity. Another standard animal model for diabetes is the db/db mouse carrying a deficient leptin receptor activity. Also this mouse is characterized by obesity, hyperglycemia and insulin resistance.

The pharmaceutical composition of the present invention comprises therapeutically effective amounts of the individual compounds and generally an acceptable pharmaceutical carrier, diluent or excipient, e.g. sterile water, physiological saline, bacteriostatic saline, i.e. saline containing about 0.9% mg/ml benzyl alcohol, phosphate-buffered saline, Hank's solution, Ringer's-lactate, lactose, dextrose, sucrose, trehalose, sorbitol, Mannitol, and the like. The composition is generally a solution or suspension. It can be administered orally, subcutaneously, intramuscularly, pulmonary, by inhalation and/or through sustained release administrations. Preferably, the composition is administered subcutaneously.

The term "therapeutically effective amount" generally means the quantity of a compound that results in the desired therapeutic and/or prophylactic effect without causing unacceptable side-effects. A typical dosage range is from about 0.01 mg per day to about 1000 mg per day. A preferred dosage range for each therapeutically effective compound is from about 0.1 mg per day to about 100 mg per day and a most preferred dosage range is from about 1.0 mg/day to about 10 mg/day, in particular about 1-5 mg/day.

In case of subsequent administration(s), the individual compounds of the pharmaceutical composition are administered during a time period where the synergistic effect of the FGF-21 compound and the GLP-1 R agonist are still measurable e.g. in a "glucose tolerance test", as e.g. shown in the Examples. The glucose tolerance test is a test to determine how quickly glucose is cleared from the blood after administration of glucose. The glucose is most often given orally ("oral glucose tolerance test" or "OGTT"). The time period for the subsequent administration of the individual compounds, in particular of the FGF-21 compound and the GLP-1 R agonist, is usually within one hour, preferably, within half an hour, most preferably within 15 minutes, in particular within 5 minutes.

Generally, the application of the pharmaceutical composition to a patient is one or several times per day, or one or several times a week, or even during longer time periods as the case may be. The most preferred application of the pharmaceutical composition of the present invention is a subcutaneous application one to three times per day in a combined dose.

"Metabolic Syndroms" as used herein, refer to medical disorders which increase the risk of developing cardiovascular diseases and/or diabetes mellitus. Medical disorders increasing the risk of developing cardiovascular diseases and/or diabetes mellitus include but are not limited to dyslipidemia, fatty liver disease (FLD), dysglycemia, impaired glucose tolerance (IGT), obesity and/or adipositas

Cardiovascular diseases are known in the art as a class of diseases that involve the heart or blood vessels (arteries and veins) such as but not limited to atherosclerosis.

Dyslipidemia is a condition wherein an abnormal amount of lipids (e.g. cholesterol, especially ldl cholesterol and/or fat such as triglycerides) is present in the blood. In developed countries, most dyslipidemias are hyperlipidemias; i.e. an elevation of lipids (e.g. triglycerides and/or ldl cholesterol) in the blood, often caused by diet and lifestyle. The prolonged elevation of insulin levels can also lead to dyslipidemia.

Fatty liver disease (FLD) is a reversible condition wherein large vacuoles of triglyceride fat accumulate in liver cells due to steatosis (i.e. abnormal retention of lipids within cells). FLD may have multiple causes however; predominately it is associated with excessive alcohol intake and obesity (with or without effects of insulin resistance).

Dysglycemia refers to an imbalance in the sugar metabolism/energy production mechanisms of the body. Diabetes mellitus is a metabolic disorder characterized by the presence of hyperglycemia. Impaired glucose tolerance (IGT) is a pre-diabetic state of dysglycemia that is associated with insulin resistance and increased risk of cardiovascular pathology and may precede type 2 diabetes mellitus by many years. Obesity is a medical condition in which excess body fat has accumulated to the extent that it may have an adverse effect on health, leading to reduced life expectancy and/or increased health problems.

The pharmaceutical composition of the present invention comprising at least a FGF-21-compound and an GLP1 Receptor agonist or comprising at least a FGF-21 compound and a DPP IV inhibitor lowers blood glucose levels up to normo-glycaemic levels and increase energy expenditure by faster and more efficient glucose utilization, and thus is useful for treating at least one metabolic syndrome and/or atherosclerosis, in particular Type 1 or Type 2 diabetes, dyslipidemia, fatty liver disease (FLD), dysglycemia, impaired glucose tolerance (IGT), obesity and/or adipositas, in particular Type 2-diabetes.

Consequently, the present invention is also directed to the use of the described pharmaceutical composition(s) for the preparation of a medicament for treating at least one of the above-mentioned diseases or disorders, and to a method for treating at least one of the above-mentioned diseases in a patient. The patient is especially selected from a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. The medicament can be prepared by methods known to a person skilled in the art, e.g. by mixing the pharmaceutically effective amounts of the compound or compounds with an acceptable pharmaceutical carrier, diluent or excipient, as described above.

In further embodiments the present invention is also directed to the described pharmaceutical composition for treating diabetes, preferably Type-2 diabetes, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

The present invention is also directed to the described pharmaceutical composition for lowering plasma glucose level, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

Furthermore, the present invention is directed to the described pharmaceutical composition for increasing the glucose tolerance, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

The present invention is also directed to the above described pharmaceutical composition for decreasing insulin tolerance, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

In further embodiments the present invention is directed to the described pharmaceutical composition for increasing the body temperature, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

In further embodiments the present invention is directed to the described pharmaceutical composition for reducing weight, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

In further embodiments the present invention is directed to the described pharmaceutical composition for decreasing the lipid content of the liver, especially the triglyceride level, preferably in a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

In further embodiments the present invention is directed to a method of treating diabetes, preferably Type-2 diabetes, of lowering plasma glucose level, especially lowering elevated plasma glucose level, of normalizing plasma glucose level, of lowering the lipid content in the liver, of lowering the plasma lipid level, especially an elevated plasma lipid level such as an elevated level of triglycerides or an elevated level of ldl cholesterol, of treating dyslipidemia (preferably hyperlipidemia), of reducing body weight, of increasing the glucose tolerance, of decreasing insulin tolerance, of increasing the body temperature, or treating obesity, of treating impaired glucose tolerance, of treating fatty liver disease of treating hyperglycemia and/or of reducing weight comprising the administration of the above described pharmaceutical composition.

Preferably, the pharmaceutical composition is administered to a diabetic patient, more preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient. Typically, the patient is a mammal, preferably a human being.

In preferred embodiments a therapeutically effective amount is administered. Typically, the dosage range is from about 0.01 mg per day to about 1000 mg per day. Preferably, the dosage range for each therapeutically effective compound is from about 0.1 mg per day to about 100 mg per day, a more preferred dosage range is from about 1.0 mg/day to about 10 mg/day, most preferably about 1-5 mg/day.

Typically, the pharmaceutical composition is administered orally, subcutaneously, intramuscularly, pulmonary, by inhalation and/or through sustained release administrations. It is preferred that the composition is administered subcutaneously.

The following aspects are also encompassed by the present invention:
1. A pharmaceutical composition comprising at least one FGF-21 (fibroblast growth factor 21) compound and at least one GLP-1 R (glucagon-like peptide-1 receptor) agonist.
2. The pharmaceutical composition of aspect 1, wherein the composition further comprises at least one anti-diabetic drug and/or at least one DPP-4 (dipeptidyl peptidase-4) inhibitor.
3. A pharmaceutical composition comprising at least one FGF-21 (fibroblast growth factor 21) compound and at least one DPP-4 (dipeptidyl peptidase-4) inhibitor.
4. The pharmaceutical composition of aspect 3, wherein the composition further comprises at least one anti-diabetic drug and/or at least one GLP1R (glucagon-like peptide-1 receptor) agonist.
5. The pharmaceutical composition of any of aspects 1-4, wherein the FGF-21 compound(s) optionally the GLP-1R agonist(s), optionally the anti-diabetic drug(s) and optionally the DPP-4 inhibitor are combined in one formulation or contained in several formulations.
6. The pharmaceutical composition of aspect 5, wherein the formulations of the FGF-21 compound(s), optionally the GLP-1R agonist(s), optionally the anti-diabetic drug(s) and optionally the DPP-4 inhibitor are suitable for simultaneous or subsequent administration(s).
7. The pharmaceutical composition of any of aspects 1-6, wherein the FGF-21 compound is selected from native FGF-21 or a FGF-21 mimetic.
8. The pharmaceutical composition of aspect 7, wherein the FGF-21 mimetic is selected from a protein having at least about 96% amino acid sequence identity to the amino acid sequence shown in SEQ ID NO: 1 and having FGF-21 activity, a FGF-21 fusion protein and/or a FGF-21 conjugate.
9. The pharmaceutical composition of aspect 8, wherein the FGF-21 mimetic is selected from a FGF-21 mutein, a FGF-21-Fc fusion protein, a FGF-21-HSA fusion protein and/or a PEGylated FGF-21.
10. The pharmaceutical composition of any of aspects 1-9, wherein the GLP-1R agonist is selected from a bioactive GLP-1, a GLP-1 analogue or a GLP-1 substitute.
11. The pharmaceutical composition of aspect 10, wherein the GLP-1R agonist is selected from GLP-1 (7-37), GLP-1 (7-36)amide, extendin-4, liraglutide, CJC-1131, albugon, albiglutide, exenatide, exenatide-LAR, oxyntomodulin, lixisenatide, geniproside, *AVE-0010* (SEQ ID NO: 9), a short peptide with GLP-1R agonistic activity and/or a small organic compound with GLP-1 R agonistic activity.
12. The pharmaceutical composition of any of aspects 1-11, wherein the anti-diabetic drug is selected from metformin, a thiazolidinedione, a sulphonylurea, and/or insulin.
13. The pharmaceutical composition of any of aspects 1-11, wherein the DPP-4 inhibitor is selected from sitagliptin, vildagliptin, saxagliptin, linagliptin, adogliptin and/or berberine.
14. Use of a pharmaceutical composition of any of aspects 1-13 for the preparation of a medicament for treating a cardiovascular disease and/or diabetes mellitus and/or at least one metabolic syndrome which increases the risk of developing cardiovascular diseases and/or diabetes mellitus in a patient.
15. The use of aspect 17, wherein the metabolic syndrome is selected from, dyslipidemia, fatty liver disease (FLD), dysglycemia, impaired glucose tolerance (IGT), obesity and/or adipositas, in particular Type 2-diabetes.
16. The use of aspect 17, wherein the cardiovascular disease is atherosclerosis.
17. The use of any of aspects 17 to 19, wherein the patient is selected from a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.
18. The pharmaceutical composition of any of aspects 1-13 for treating a cardiovascular disease and/or diabetes mellitus and/or at least one metabolic syndrome which increases the risk of developing a cardiovascular disease and/or diabetes mellitus, preferably Type 2-diabetes.
19. The pharmaceutical composition of aspect 18, wherein the metabolic syndrome is selected from dyslipidemia, fatty liver disease (FLD), dysglycemia, impaired glucose tolerance (IGT), obesity and/or adipositas.
20. The pharmaceutical composition of aspect 18, wherein the cardiovascular disease is atherosclerosis.
21. The pharmaceutical composition of any of the aspects 1-13 for lowering plasma glucose level, for lowering the lipid content in the liver, for treating hyperlipidemia, for treating hyperglycemia, for increasing the glucose tolerance, for decreasing insulin tolerance, for increasing the body temperature, and/or for reducing weight.
22. The pharmaceutical composition of aspect 21, wherein the plasma glucose level is lowered, the lipid content in the liver is lowered, the glucose tolerance is increased, the insulin tolerance is increased, the body temperature is increased, and/or the weight is reduced in a diabetic patient, preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.
23. The pharmaceutical composition of aspect 22, wherein the patient is a mammal, preferably a human being.
24. Use of a pharmaceutical composition of any of aspects 1-13 for the preparation of a medicament for lowering plasma glucose level, for lowering the lipid content in the liver, for increasing the glucose tolerance, for decreasing insulin tolerance, for increasing the body temperature, and/or for reducing weight.
25. A method of treating a cardiovascular disease and/or diabetes mellitus and/or at least one metabolic syndrome which increases the risk of developing a cardiovascular disease and/or diabetes mellitus, preferably Type 2-diabetes comprising the administration of a pharmaceutical composition of any of the aspects 1-13.
26. The method of aspect 25, wherein the metabolic syndrome is selected from dyslipidemia, fatty liver disease (FLD), dysglycemia, impaired glucose tolerance (IGT), obesity and/or adipositas.
27. The method of aspect 26, wherein the cardiovascular disease is atherosclerosis.
28. A method of treating of lowering plasma glucose level, of lowering the lipid content in the liver, of treating hyperlipidemia, of treating hyperglycemia, of increasing the glucose tolerance, of decreasing insulin tolerance, of increasing the body temperature, and/or of reducing weight comprising the administration of a pharmaceutical composition of any of the aspects 1-13.
29. The method of any of aspects 25-28, wherein the pharmaceutical composition is administered to a diabetic patient, preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.
30. The method of aspect 29, wherein the patient is a mammal, preferably a human being.
31. The method of any of aspects 25 to 30, wherein a therapeutically effective amount is administered.
32. The method of any of aspects 25 to 31, wherein the pharmaceutical composition is administered in a dosage range of 0.01 mg per day to about 1000 mg per day, preferably about 0.1 mg per day to about 100 mg per day, more preferably about 1.0 mg/day to about 10 mg/day, most preferably about 1-5 mg/day.
33. The method of any of aspects 25 to 32, wherein the pharmaceutical composition is administered orally, subcutaneously, intramuscularly, pulmonary, by inhalation and/or through sustained release administrations, preferably, the composition is administered subcutaneously.

The following figures and examples are for the purpose of illustration only and are not intended to be limiting of the present invention.

### Figures

- Fig. 1: shows the chemical structure of liraglutide.
- Fig. 2: shows the chemical structure of CJC-1131.
- Fig. 3: shows the chemical structure of Formula I: (R)-4-oxo-4-[3-(R1)-5,6-dihydro[1,2,4]tri-azolo[4,3-a]pyrazin-7(8H)-yl]-1-(2-R2,4-R3,5-R4phenyl)butan-2-amine, with R1 being H or Halogen: F, Cl, Br, J, At; CF₃, or CH₂CF₃; R2 being H or Halogen: F, Cl, Br, J, At, R3 being H or Halogen: F, Cl, Br, J, At; R4 being H or Halogen: F, Cl, Br, J, At
- Fig. 4: shows the plasma glucose levels over time after subcutaneous injection of FGF-21 together with AVE0010 in *ob*/*ob* mice. All data are means±SEM, n = 6 per group.
- Fig. 5: shows the results of an oral glucose tolerance test (OGTT) after ten days subcutaneous injection of FGF-21 together with AVE0010 in *ob*/*ob* mice. All data are means±SEM, n = 6 per group.
- Fig. 6: shows the liver triglyceride levels after ten days subcutaneous injection of FGF-21 together with oral treatment of sitagliptin in *ob*/*ob* mice. All data are means±SEM, n = 6 per group, * P < 0.05; *** P < 0.001 vs. vehicle-treated obese control.
- Fig. 7: shows the plasma glucose levels over time after subcutaneous injection of FGF-21 together with AVE0010 in *dbldb* mice. All data are means±SEM, n = 6 per group.
- Fig. 8: shows the results of an OGTT after twenty-one days subcutaneous injection of FGF-21 together with AVE0010 in *dbldb* mice. All data are means±SEM, n = 6 per group.
- Fig.9: shows the plasma glucose levels over a period of eight weeks after subcutaneous injection of FGF-21 together with AVE001 0 or exenatide in *dbldb* mice. All data are means±SEM, n = 6 per group.
- Fig. 10: shows the plasma HbA1c levels over a period of eight weeks after subcutaneous injection of FGF-21 together with AVE001 0 or exenatide in *dbldb* mice. All data are means±SEM, n = 6 per group.
- Fig. 11: shows the body temperatures of *dbldb* mice after eight weeks subcutaneous injection of FGF-21 together with AVE0010 or exenatide. All data are means±SEM, n = 6 per group, ** P < 0.01; *** P < 0.001 vs. vehicle-treated obese control.
- Fig. 12: shows the results of an insulin tolerance test (ITT) after 16 days subcutaneous injection of FGF-21 together with AVE0010 in DIO mice. All data are means±SEM, n = 8 per group, * P < 0.05 vs. vehicle-treated HFD control.
- Fig. 13: shows the weight change in DIO mice after 3 weeks subcutaneous injection of FGF-21 together with AVE0010. All data are means±SEM, n = 8 per group, * P < 0.05 vs. vehicle-treated HFD control.

### Examples

### 1. Treatment of ob/ob mice

Female *ob*/*ob* mice (B6.V-LEP OB/J, age of 6 weeks) were obtained from Charles Rivers Laboratories (Sulzfeld, Germany). Mice were randomly assigned to treatment or vehicle groups, and the randomization was stratified by body weight and fed blood glucose levels. The animals were housed in groups of 6 at 23°C and on a 12 h light-dark cycle. All experimental procedures were conducted according to German Animal Protection Law. Mice were fed *ad libitum* with standard rodent chow during the drug treatment periods. Body weight was recorded every other day, and food intake was measured once a week throughout the studies.

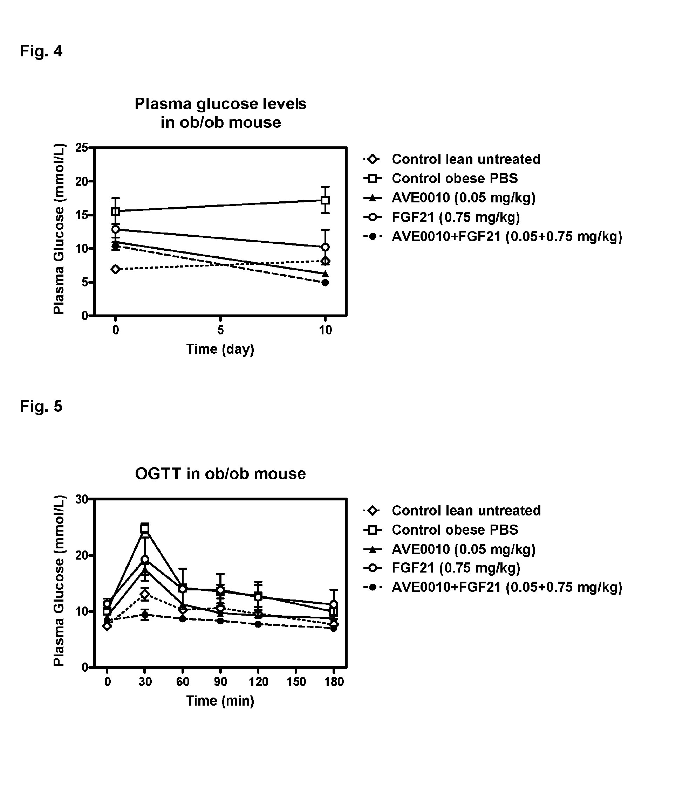

*Ob*/*ob* mice were treated with vehicle (PBS), 0.05 mg · kg⁻¹ · day⁻¹ AVE0010, 0.75 mg · kg⁻¹ · day⁻¹ recombinant human FGF-21 (SEQ ID NO: 2) or a combined dose of FGF-21 (SEQ ID NO: 2) and AVE0010 (0.75 + 0.05 mg · kg⁻¹ · day⁻¹ subcutaneously once daily. One day before the first treatment and at study day 10 blood glucose was measured by tail tip bleeding under fed conditions. As shown in Figure 3 the blood glucose levels of the treated mice became normo-glycaemic. On study day 8 a glucose tolerance test (OGTT) was performed. Fasted mice were orally challenged with 2 g · kg⁻¹ glucose. Blood glucose was measured at indicated time points by tail tip bleeding without anaesthesia. The results of the OGTT are shown in Figure 4. Compared to the administration of only FGF-21 or only AVE0010 glucose tolerance was markedly stronger improved by combination treatment. The combination treated obese animals were even more glucose tolerant than lean control animals.

In another experiment female *ob*/*ob* mice were treated with vehicle (PBS) or 0.75 mg · kg⁻¹ · day⁻¹ recombinant human FGF-21 (SEQ ID NO: 2) subcutaneously or 5 mg · kg⁻¹ · day⁻¹ sitagliptin per os once daily, or a combined dose of FGF-21 s.c. (SEQ ID NO: 2) and sitagliptin p.o. (0.75 + 5 mg · kg⁻¹ · day⁻¹. At day 10 livers were collected for lipid analysis. Hepatic lipid extraction was conducted and lipid contents were measured.

In livers of ob/ob mice treated for ten days with a subcutaneous injection of FGF-21 in combination with an oral administration of the DPPIV inhibitor sitagliptin a markedly reduced lipid accumulation was found when compared with vehicle-treated mice or administration of FGF-21 or sitagliptin alone (Figure 5).

### 2. Treatment of db/db mice

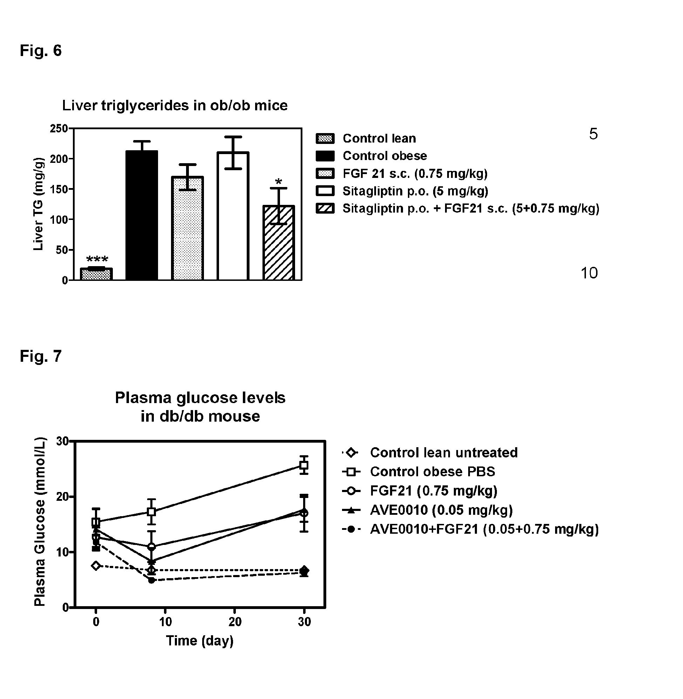

Female *dbldb* mice (BKS.Cg-m +/+ Leprdb /J, age of 6 weeks) were treated with vehicle (PBS), 0.05 mg · kg⁻¹ · day⁻¹ AVE0010, 0.75 mg · kg⁻¹ · day⁻¹ recombinant human FGF-21 (SEQ ID NO: 2) or a combined dose of FGF-21 (SEQ ID NO: 2) and AVE0010 (0.75 + 0.05 mg · kg⁻¹ · day⁻¹) subcutaneously once daily. Mice were fed *ad libitum.* Before the first treatment, after one week and 4 weeks blood glucose and HbA1 c were measured under fed conditions. After 21 days of treatment an oral glucose tolerance test (OGTT) was initiated. Fasted mice were orally challenged with 2 g · kg⁻¹ glucose solution and blood glucose was measured at indicated time points. The results are shown in Figure 6 and 7. The administration of the FGF21 plus AVE0010 combination results in normalisation of blood glucose and improved dramatically the glucose tolerance compared to the vehicle treated obese control. On the other hand leads the single treatment of FGF21 or AVE001 0 compared to the combination only to inhibition of blood glucose increase and a small improvement in glucose tolerance.

In another experiment female *dbldb* mice (BKS.Cg-m +/+ Leprdb /J, age of 12 weeks) were treated for 8 weeks with vehicle (PBS), or 0.05 mg · kg⁻¹ · day⁻¹ AVE0010, or 0.04 mg · kg⁻¹ · day⁻¹ exenatide, or 0.75 mg · kg⁻¹ · day⁻¹ recombinant human FGF-21 (SEQ ID NO: 2), or a combined dose of FGF-21 (SEQ ID NO: 2) and AVE0010 (0.75 + 0.05 mg · kg⁻¹ · day⁻¹), or a combined dose of FGF-21 (SEQ ID NO: 2) and exenatide (0.75 + 0.04 mg · kg⁻¹ · day⁻¹) subcutaneously once daily. Blood glucose levels and HbA1c were analysed under fed conditions. Rectal body temperatures were measured after 8 weeks of treatment.

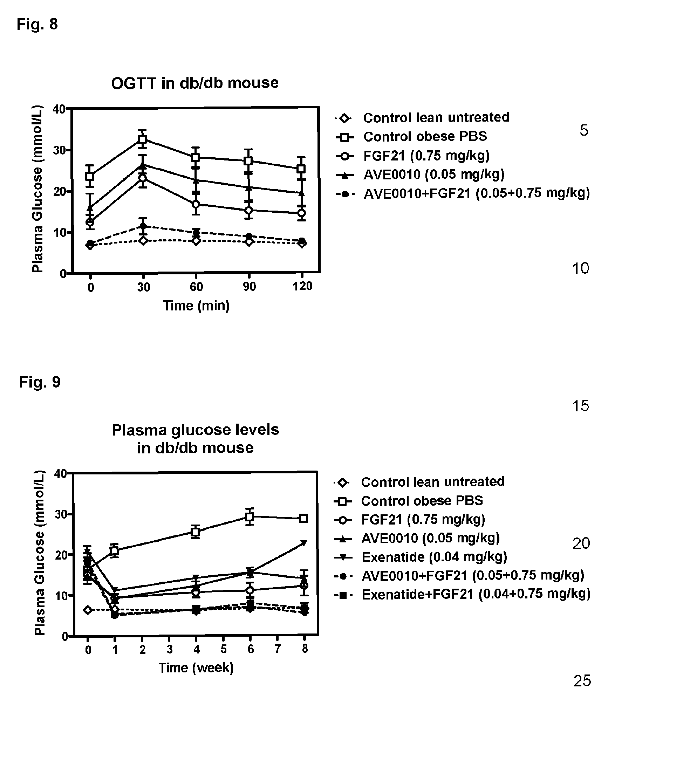

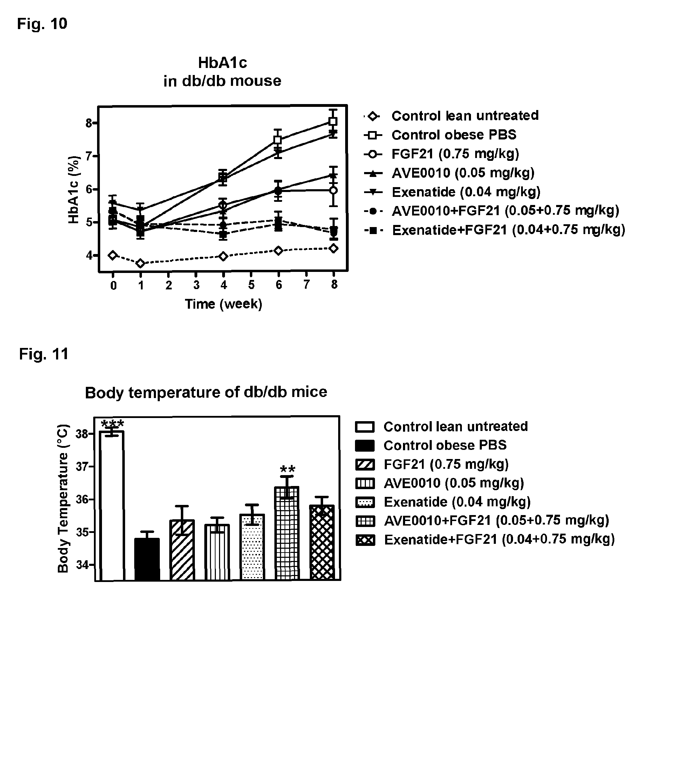

Both GLP1-R agonists AVE0010 and exenatide totally normalized blood glucose levels and stopped further increase of HbA1c when combined with FGF-21 whereas single treatment were not that effective, results are shown in Figure 8 and Figure 9. An increase in body temperature of 1-1.6°C towards physiological normal value of lean control animals was the result of combined treatment with FGF-21 plus AVE0010 or exenatide in *dbldb* mice, single treatment was less effective as shown in Figure 10.

### 3. Treatment of DIO mice

In another experiment female C57bl6 mice received 22 weeks a high fat diet to induce obesity (DIO). There after the mice were treated for 3 weeks with vehicle (PBS), or 0.05 mg · kg⁻¹ · day⁻¹ AVE0010, or 0.75 mg · kg⁻¹ · day⁻¹ recombinant human FGF-21 (SEQ ID NO: 2), or a combined dose of FGF-21 (SEQ ID NO: 2) and AVE0010 (0.75 + 0.05 mg · kg⁻¹ · day⁻¹). Body weight was recorded every other day, and food intake was measured once a week throughout the study.

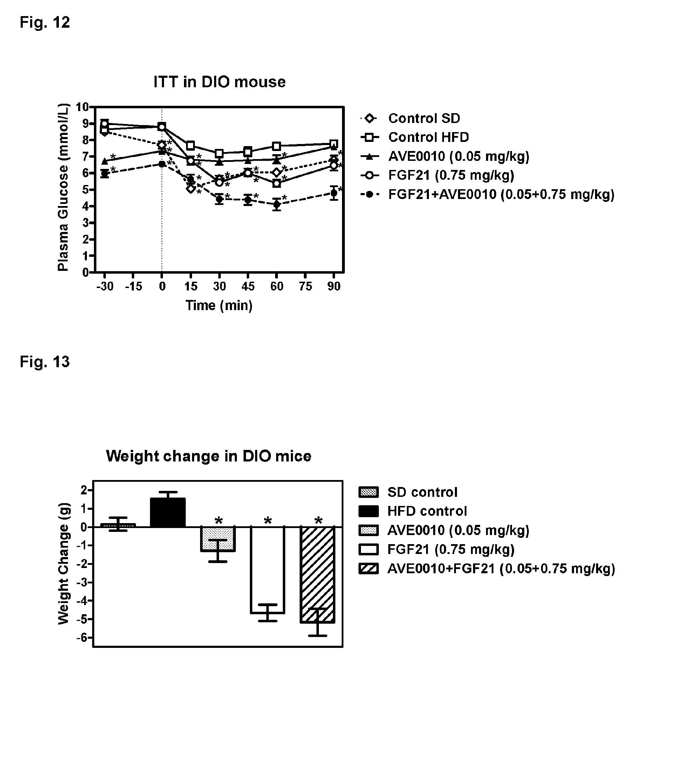

Insulin sensitivity was analysed by performing an insulin tolerance test (ITT) in non-fasted animals after 16 days treatment. Insulin (0.75 units · kg⁻¹) was injected i.p., and blood glucose was measured at the indicated time points (Figure 11). Serum insulin was subsequently measured by mouse Insulin ELISA kit (Mercodia, Uppsala, Sweden). Mice treated for 16 days a combination of FGF-21 and AVE0010 showed significantly improved insulin sensitivity and decreased basal blood glucose levels under fed conditions than mice receiving vehicle or single treatment. A dramatic decrease of body weight compared to vehicle or single treated mice were observed as shown in Figure 12.

### Sequences

**Human FGF-21 (SEQ ID NO: 1):** 
MDSDETGFEHSGLWVSVLAGLLLGACQAHPIPDSSPLLQPGGQVRQRYLYTDDAQQTEAHLEIREDGT
VGGAADQSPESLLQLKALKPGVIQILGVKTSRFLCQRPDGALYGSLHFDPEACSFRELLLEDGYNVYQ
SEAHGLPLHLPGNKSPHRDPAPRGPARFLPLPGLPPAPPEPPGILAPQPPDVGSSDPLSMVGPSQGRS
PSYAS
 **Mutated FGF-21 (G + FGF21 H29-S209; SEQ ID NO: 2):** 
GHPIPDSSPLLQFGGQVRQRYLYTDDAQQTEAHLEIREDGTVGGAADQSPESLLQLKALKPGVIQILG
VKTSRFLCQRPDGALYGSLHFDPEACSFRELLLEDGYNVYQSEAHGLPLHLPGNKSPHRDPAPRGPAR
FLPLPGLPPAPPEPPGILAPQPPDVGSSDPLSMVGPSQGRSPSYAS
 **Human GLP-1(7-37) (SEQ ID NO: 3):** HAEGTFTSDVSSYLEGQAAKEFIAWLVKGRG-NH₂ **Human GLP-1(7-36)NH₂ (SEQ ID NO: 4):** HAEGTFTSDVSSYLEGQAAKEFIAWLVKGR-NH₂ **Exendin-4 (SEQ ID NO: 5):** HGEGTFTSDLSKQMEEEAVRLFIEWLKNGGPSSGAPPPS-NH₂ **Exenatide (SEQ ID NO: 6):** HGEGTFTSDLSKQMEEEAVRLFIETLKNGGPSSGAPPPS-NH₂ **Oxyntomodulin (SEQ ID NO: 7):** HSQGTFTSDYSKYLDSRRAQDFVQWLMNTKRNRNNIA-NH₂ **Lixisenatide (SEQ ID NO: 8)** HGEGTFTSDLSKQMEEEAVRLFTEWLKNGGPSSGAPPSKKKKKK-NH2 ***AVE0010* (SEQ ID NO: 9):** HGEGTFTSDLSKQMEEEAVRLFIEWLKNGGPSSGAPPSKKKKKK-NH2

## Claims

1. A pharmaceutical composition comprising at least one FGF-21 (fibroblast growth factor 21) compound and at least one GLP-1 R (glucagon-like peptide-1 receptor) agonist.

2. The pharmaceutical composition of claim 1, wherein the composition further comprises at least one anti-diabetic drug and/or at least one DPP-4 (dipeptidyl peptidase-4) inhibitor.

3. A pharmaceutical composition comprising at least one FGF-21 (fibroblast growth factor 21) compound and at least one DPP-4 (dipeptidyl peptidase-4) inhibitor.

4. The pharmaceutical composition of claim 3, wherein the composition further comprises at least one anti-diabetic drug and/or at least one GLP1 R (glucagon-like peptide-1 receptor) agonist.

5. The pharmaceutical composition of any of claims 1-4, wherein the FGF-21 compound(s) optionally the GLP-1R agonist(s), optionally the anti-diabetic drug(s) and optionally the DPP-4 inhibitor are combined in one formulation or contained in several formulations.

6. The pharmaceutical composition of claim 5, wherein the formulations of the FGF-21 compound(s), optionally the GLP-1R agonist(s), optionally the anti-diabetic drug(s) and optionally the DPP-4 inhibitor are suitable for simultaneous or subsequent administration(s).

7. The pharmaceutical composition of any of claims 1-6, wherein the FGF-21 compound is selected from native FGF-21 or a FGF-21 mimetic.

8. The pharmaceutical composition of claim 7, wherein the FGF-21 mimetic is selected from a protein having at least about 96% amino acid sequence identity to the amino acid sequence shown in SEQ ID NO: 1 and having FGF-21 activity, a FGF-21 fusion protein and/or a FGF-21 conjugate.

9. The pharmaceutical composition of claim 8, wherein the FGF-21 mimetic is selected from a FGF-21 mutein, a FGF-21-Fc fusion protein, a FGF-21-HSA fusion protein and/or a PEGylated FGF-21.

10. The pharmaceutical composition of any of claims 1-9, wherein the GLP-1R agonist is selected from a bioactive GLP-1, a GLP-1 analogue or a GLP-1 substitute.

11. The pharmaceutical composition of claim 10, wherein the GLP-1R agonist is selected from GLP-1 (7-37), GLP-1 (7-36)amide, extendin-4, liraglutide, CJC-1131, albugon, albiglutide, exenatide, exenatide-LAR, oxyntomodulin, lixisenatide, geniproside, *AVE-0010* (SEQ ID NO: 9), a short peptide with GLP-1R agonistic activity and/or a small organic compound with GLP-1 R agonistic activity.

12. The pharmaceutical composition of any of claims 1-11, wherein the anti-diabetic drug is selected from metformin, a thiazolidinedione, a sulphonylurea, and/or insulin.

13. The pharmaceutical composition of any of claims 1-11, wherein the DPP-4 inhibitor is selected from sitagliptin, vildagliptin, saxagliptin, linagliptin, adogliptin and/or berberine.

14. Use of a pharmaceutical composition of any of claims 1-13 for the preparation of a medicament for treating a cardiovascular disease and/or diabetes mellitus and/or at least one metabolic syndrome which increases the risk of developing cardiovascular diseases and/or diabetes mellitus in a patient.

15. The use of claim 17, wherein the metabolic syndrome is selected from, dyslipidemia, fatty liver disease (FLD), dysglycemia, impaired glucose tolerance (IGT), obesity and/or adipositas, in particular Type 2-diabetes.

16. The use of claim 17, wherein the cardiovascular disease is atherosclerosis.

17. The use of any of claims 17 to 19, wherein the patient is selected from a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.

18. The pharmaceutical composition of any of claims 1-13 for treating a cardiovascular disease and/or diabetes mellitus and/or at least one metabolic syndrome which increases the risk of developing a cardiovascular disease and/or diabetes mellitus, preferably Type 2-diabetes.

19. The pharmaceutical composition of claim 18, wherein the metabolic syndrome is selected from dyslipidemia, fatty liver disease (FLD), dysglycemia, impaired glucose tolerance (IGT), obesity and/or adipositas.

20. The pharmaceutical composition of claim 18, wherein the cardiovascular disease is atherosclerosis.

21. The pharmaceutical composition of any of the claims 1-13 for lowering plasma glucose level, for lowering the lipid content in the liver, for treating hyperllipidemia, for treating hyperglycemia, for increasing the glucose tolerance, for decreasing insulin tolerance, for increasing the body temperature, and/or for reducing weight.

22. The pharmaceutical composition of claim 21, wherein the plasma glucose level are lowered, the lipid content in the liver is lowered, the glucose tolerance is increased, the insulin tolerance is increased, the body temperature is increased, and/or the weight is reduced in a diabetic patient, preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.

23. The pharmaceutical composition of claim 22, wherein the patient is a mammal, preferably a human being.

24. Use of a pharmaceutical composition of any of claims 1-13 for the preparation of a medicament for lowering plasma glucose level, for lowering the lipid content in the liver, for increasing the glucose tolerance, for decreasing insulin tolerance, for increasing the body temperature, and/or for reducing weight.

25. A method of treating a cardiovascular disease and/or diabetes mellitus and/or at least one metabolic syndrome which increases the risk of developing a cardiovascular disease and/or diabetes mellitus, preferably Type 2-diabetes comprising the administration of a pharmaceutical composition of any of the claims 1-13.

26. The method of claim 25, wherein the metabolic syndrome is selected from dyslipidemia, fatty liver disease (FLD), dysglycemia, impaired glucose tolerance (IGT), obesity and/or adipositas.

27. The method of claim 26, wherein the cardiovascular disease is atherosclerosis.

28. A method of treating of lowering plasma glucose level, of lowering the lipid content in the liver, of treating hyperlipidemia, of treating hyperglycemia, of increasing the glucose tolerance, of decreasing insulin tolerance, of increasing the body temperature, and/or of reducing weight comprising the administration of a pharmaceutical composition of any of the claims 1-13.

29. The method of any of claims 25-28, wherein the pharmaceutical composition is administered to a diabetic patient, preferably selected from the group consisting of a Type 1-diabetic patient, a Type 2-diabetic patient, in particular a diet-treated Type 2-diabetic patient, a sulfonylurea-treated Type 2-diabetic patient, a far-advanced stage Type 2-diabetic patient and/or a long-term insulin-treated Type 2-diabetic patient.

30. The method of claim 29, wherein the patient is a mammal, preferably a human being.

31. The method of any of claims 25 to 30, wherein a therapeutically effective amount is administered.

32. The method of any of claims 25 to 31, wherein the pharmaceutical composition is administered in a dosage range of 0.01 mg per day to about 1000 mg per day, preferably about 0.1 mg per day to about 100 mg per day, more preferably about 1.0 mg/day to about 10 mg/day, most preferably about 1-5 mg/day.

33. The method of any of claims 25 to 32, wherein the pharmaceutical composition is administered orally, subcutaneously, intramuscularly, pulmonary, by inhalation and/or through sustained release administrations, preferably, the composition is administered subcutaneously.


SEQUENCE LISTING

<110>  SANOFI

<120>  Pharmaceutical composition for treating a metabolic syndrome

<130>  DE2011/168

<160>  9

<170>  PatentIn version 3.3

<210>  1
<211>  209
<212>  PRT
<213>  Homo sapiens

<400>  1

Met Asp Ser Asp Glu Thr Gly Phe Glu His Ser Gly Leu Trp Val Ser
1               5                   10                  15

Val Leu Ala Gly Leu Leu Leu Gly Ala Cys Gln Ala His Pro Ile Pro
            20                  25                  30

Asp Ser Ser Pro Leu Leu Gln Pro Gly Gly Gln Val Arg Gln Arg Tyr
        35                  40                  45

Leu Tyr Thr Asp Asp Ala Gln Gln Thr Glu Ala His Leu Glu Ile Arg
    50                  55                  60

Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95

Lys Thr Ser Arg Phe Leu Cys Gln Arg Pro Asp Gly Ala Leu Tyr Gly
            100                 105                 110

Ser Leu His Phe Asp Pro Glu Ala Cys Ser Phe Arg Glu Leu Leu Leu
        115                 120                 125

Glu Asp Gly Tyr Asn Val Tyr Gln Ser Glu Ala His Gly Leu Pro Leu
    130                 135                 140

His Leu Pro Gly Asn Lys Ser Pro His Arg Asp Pro Ala Pro Arg Gly
145                 150                 155                 160

Pro Ala Arg Phe Leu Pro Leu Pro Gly Leu Pro Pro Ala Pro Pro Glu
                165                 170                 175

Pro Pro Gly Ile Leu Ala Pro Gln Pro Pro Asp Val Gly Ser Ser Asp
            180                 185                 190

Pro Leu Ser Met Val Gly Pro Ser Gln Gly Arg Ser Pro Ser Tyr Ala
        195                 200                 205

Ser

<210> 2
<211> 182
<212> PRT
<213> unknown

<220>
<223> G linked to fragment of human FGF21 from H29 to S209

<400> 2

Gly His Pro Ile Pro Asp Ser Ser Pro Leu Leu Gln Phe Gly Gly Gln
1               5                   10                  15

Val Arg Gln Arg Tyr Leu Tyr Thr Asp Asp Ala Gln Gln Thr Glu Ala
            20                  25                  30

His Leu Glu Ile Arg Glu Asp Gly Thr Val Gly Gly Ala Ala Asp Gln
        35                  40                  45

Ser Pro Glu Ser Leu Leu Gln Leu Lys Ala Leu Lys Pro Gly Val Ile
    50                  55                  60

Gln Ile Leu Gly Val Lys Thr Ser Arg Phe Leu Cys Gln Arg Pro Asp
65                  70                  75                  80

Gly Ala Leu Tyr Gly Ser Leu His Phe Asp Pro Glu Ala Cys Ser Phe
                85                  90                  95

Arg Glu Leu Leu Leu Glu Asp Gly Tyr Asn Val Tyr Gln Ser Glu Ala
            100                 105                 110

His Gly Leu Pro Leu His Leu Pro Gly Asn Lys Ser Pro His Arg Asp
        115                 120                 125

Pro Ala Pro Arg Gly Pro Ala Arg Phe Leu Pro Leu Pro Gly Leu Pro
    130                 135                 140

Pro Ala Pro Pro Glu Pro Pro Gly Ile Leu Ala Pro Gln Pro Pro Asp
145                 150                 155                 160

Val Gly Ser Ser Asp Pro Leu Ser Met Val Gly Pro Ser Gln Gly Arg
                165                 170                 175

Ser Pro Ser Tyr Ala Ser
            180

<210> 3
<211> 31
<212> PRT
<213> unknown

<220>
<223>  Fragment of human GLP-1 (7 to 37)


<220>
<221>  MOD_RES
<222>  (31)..(31)
<223>  AMIDATION

<400>  3

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  4
<211>  30
<212>  PRT
<213>  unknown

<220>
<223>  fragment of human GLP-1 (7-36)


<220>
<221>  MOD_RES
<222>  (30)..(30)
<223>  AMIDATION

<400>  4

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg
            20                  25                  30


<210>  5
<211>  39
<212>  PRT
<213>  Heloderma suspectum


<220>
<221>  MOD_RES
<222>  (39)..(39)
<223>  AMIDATION

<400>  5

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
        35


<210>  6

<211>  39
<212>  PRT
<213>  Heloderma suspectum

<220>
<221>  MOD_RES
<222>  (39)..(39)
<223>  AMIDATION

<400>  6

His Gly Glu Gly Thr Phe Thr Ser Asp Leu Ser Lys Gln Met Glu Glu
1               5                   10                  15

Glu Ala Val Arg Leu Phe Ile Glu Thr Leu Lys Asn Gly Gly Pro Ser
            20                  25                  30

Ser Gly Ala Pro Pro Pro Ser
        35

<210>  7
<211>  37
<212>  PRT
<213>  Homo sapiens

<220>
<221>  MOD_RES
<222>  (37)..(37)
<223>  AMIDATION

<400>  7

His Ser Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Lys Arg Asn
            20                  25                  30

Arg Asn Asn Ile Ala
        35

<210>  8
<211>  44
<212>  PRT
<213>  unknown

<220>
<223>  GLP-1 like peptide

<220>
<221>  MOD_RES
<222>  (44)..(44)

<400>  8

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser

20                  25                  30

Ser Gly Ala Pro Pro Ser Lys Lys Lys Lys Lys Lys
        35                  40

<210>  9
<211>  44
<212>  PRT
<213>  unknown

<220>
<223>  GLP-1 like peptide

<220>
<221>  MOD_RES
<222>  (44)..(44)
<223>  AMIDATION

<400>  9

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Ser
            20                  25                  30

Ser Gly Ala Pro Pro Ser Lys Lys Lys Lys Lys Lys
        35                  40